# EUROPEAN PATENT SPECIFICATION

(11) **EP 2 351 569 B1**
(45) Date of publication and mention of the grant of the patent: **22.08.2012**
(21) Application number: 11000632.7
(22) Date of filing: 25.10.2006
(51) Int. Cl.: A61K 31/551, A61K 31/192, A61K 31/343, A61K 31/4409, A61K 31/506, A61K 31/519, A61K 31/5578, A61P 9/08, A61P 9/10, A61P 9/12, A61K 31/52

(54) **Fasudil in combination therapies for the treatment of pulmonary arterial hypertension**
Kombinationstherapie mit Fasudil zur Behandlung von pulmonaler Hypertonie
Fasudil dans des thérapies combinées pour le traitement de l'hypertension artérielle pulmonaire

(30) Priority: 26.10.2005 US 730273 P
(43) Date of publication of application: 03.08.2011
(62) Divisional of application: 09007232.3
(73) Proprietor: Asahi Kasei Pharma Corporation, Tokyo 101-8101 (JP)
(72) Inventor: Fong, Benson, Brisbane CA 94005-1846 (US)
(74) Representative: Forstmeyer, Dietmar

(56) References cited:
- EP-A2- 1 097 711
- GUILLUY CHRISTOPHE ET AL: "Inhibition of RhoA/Rho kinase pathway is involved in the beneficial effect of sildenafil on pulmonary hypertension.", BRITISH JOURNAL OF PHARMACOLOGY DEC 2005 LNKD- PUBMED:16205723, vol. 146, no. 7, 3 October 2005 (2005-10-03), pages 1010-1018, XP002640064, ISSN: 0007-1188
- NAGAOKA TETSUTARO ET AL: "Inhaled Rho kinase inhibitors are potent and selective vasodilators in rat pulmonary hypertension.", AMERICAN JOURNAL OF RESPIRATORY AND CRITICAL CARE MEDICINE 1 MAR 2005 LNKD- PUBMED:15563635, vol. 171, no. 5, 1 March 2005 (2005-03-01) , pages 494-499, XP002640065, ISSN: 1073-449X
- FUKUMOTO Y ET AL: "Acute vasodilator effects of a Rho-kinase inhibitor, fasudil, in patients with severe pulmonary hypertension", HEART 2005 UNITED KINGDOM, vol. 91, no. 3, March 2005 (2005-03), pages 391-392, XP002424679, ISSN: 1355-6037
- "ADVANCING THERAPY FOR PULMONARY ARTERIAL HYPERTENSION CAN ANIMAL MODELS HELP?", AMERICAN REVIEW OF RESPIRATORY DISEASE, NEW YORK, NY, US, vol. 169, no. 1, January 2004 (2004-01), pages 5-6, XP008041696, ISSN: 0003-0805
- RAJASEKARAN MAHADEVAN ET AL: "Rho-kinase inhibition improves erectile function in aging male Brown-Norway rats.", JOURNAL OF ANDROLOGY 2005 MAR-APR LNKD- PUBMED:15713824, vol. 26, no. 2, March 2005 (2005-03), pages 182-188, XP002640066, ISSN: 0196-3635

## Description

### Field of the Invention

Embodiments of this invention are related to therapeutic formulations and methods of using Rho-kinase inhibitors, namely fasudil, in combination with a PDE inhibitor namely sildenafil, for the treatment and/or prevention of PAH and/or stable angina.

### Background of the Invention

Pulmonary arterial hypertension is a debilitating disease characterized by an increase in pulmonary vascular resistance leading to right ventricular failure and death. PAH with no apparent cause is termed primary pulmonary hypertension ("PPH"). Recently, various pathophysiological changes associated with this disorder, including vasoconstriction, vascular remodeling (i.e. proliferation of both media and intima of the pulmonary resistance vessels), and *in situ* thrombosis have been characterized (e.g., D'Alonzo, G.E. et al. 1991 Ann Intern Med 115:343-349; Palevsky, H.I. et al. 1989 Circulation 80:1207-1221; Rubin, L.J. 1997 N Engl J Med 336:111-117; Wagenvoort, C.A. & Wagenvoort, N. 1970 Circulation 42:1163-1184; Wood, P. 1958 Br Heart J 20:557-570). Impairment of vascular and endothelial homeostasis is evidenced from a reduced synthesis of prostacyclin (PGI₂), increased thromboxane production, decreased formation of nitric oxide and increased synthesis of endothelin-1 (Giaid, A. & Saleh, D. 1995 N Engl J Med 333:214-221; Xue, C & Johns, R.A. 1995 N Engl J Med 333:1642-1644). The intracellular free calcium concentration of vascular smooth muscle cells of pulmonary arteries in PPH has been reported to be elevated.

The pathogenesis of pulmonary hypertension ("PH") is a complex and multifactorial process. Pathologic changes of pulmonary arteries, which involve endothelial dysfunction, endothelial and smooth muscle cell proliferation, and increased vasoconstriction, decrease the lumen area of the pulmonary microvasculature, causing fixed elevation of pulmonary vascular resistance. Although these pathological features are common to all forms of human PH, the mechanisms responsible for this abnormal vascular proliferation are unknown. However, impairment of endothelial functions leading to an imbalance of vasodilator and vasoconstrictor influences is likely to play a central role in the initiation and progression of PH. Drugs that improve the endothelial function or restore the altered balance of endothelium-derived vasoactive mediators such as endothelin-1 receptor antagonists and prostacyclin analogues are used to treat this disease with moderate success. The NO/cGMP axis is also considered as a major target for the treatment of PH. Recently, the type 5 phosphodiesterase inhibitor sildenafil has been identified as a promising therapeutic agent for PH. The type 5 phosphodiesterase is the major cGMP-degrading phosphodiesterase in the pulmonary vasculature and is upregulated in PH. Sildenafil reduces right ventricular hypertrophy in chronic hypoxic mice, pulmonary arterial pressure in chronic hypoxic rats, and improves survival rate in rats with PH induced by monocrotaline injection. Short-term studies in patients with PH suggest that sildenafil is an effective pulmonary vasodilator.

On the other hand, recent pharmacological studies have suggested a role for the serine/threonine kinase Rho kinase in the development of PH. *In vivo,* intravenous or oral treatment with Rho kinase inhibitor (Y-27632 or fasudil) nearly normalizes the high pulmonary arterial pressure in chronically hypoxic rats, attenuates the development of chronic hypoxia-induced PH in mice, and reduces pulmonary arterial lesions in the model of monocrotaline-induced PH in rats (Abe K. et al.. 2004 Circ. Res. 94:385-393.; Fagan K.A. et al.. 2004 Am. J. Physiol. Lung Cell. Mol. Physiol. 287:L656-L664; Nagaoka et al., 2004 Am. J. Physiol. Lung Cell Mol. Physiol. 287:L665-L672). In addition, inhaled Y-27632 or fasudil causes sustained and selective pulmonary vasodilation in monocrotaline-induced PH and in spontaneous PH in fawn-hooded rats, as well as in chronically hypoxic rats (Nagaoka et al. 2005 Am. J. Respir. Crit. Care Med. 171:494-499). Rho kinase is one of the main downstream effectors of the small G protein RhoA, which functions as a tightly regulated molecular switch that governs a wide range of cellular functions (Van Aelst & D'Souza-Schorey, 1997 Genes Dev. 11:2295-2322). In particular, Rho kinase phosphorylates the myosin phosphatase target subunit 1 (MYPT1) of smooth muscle myosin phosphatase at Thr-696, leading to the inhibition of its activity. This inhibition of smooth muscle myosin phosphatase activity is a primary mechanism of the Ca²⁺ sensitization of smooth muscle contraction. A large body of evidence has now been obtained regarding the important functions of RhoA in the vasculature, and RhoA has been shown to play a major role in the regulation of vascular cell processes such as actin cytoskeleton organization, contraction, gene expression, and differentiation. In vascular smooth muscle cells, RhoA has been shown to be regulated by the NO/cGMP pathways. RhoA is phosphorylated by cGMP-dependent protein kinase (PKG) (Sauzeau et al., 2000 J. Biol. Chem. 275:21722-21729). This phosphorylation prevents the translocation of active GTP-bound RhoA to the membrane, which is an obligatory step for the activation of its downstream effectors. Activation of the NO/cGMP pathway thus leads to the inhibition of RhoA-dependent functions, including actin cytoskeleton organization, Ca²⁺ sensitization of the contraction, and gene transcription (Sauzeau et al., 2000 J. Biol. Chem. 275:21722-21729; Gudi et al., 2002 J. Biol. Chem. 277:37382-37393). The beneficial effect of Rho kinase inhibitor on PH could be ascribed to multiple mechanisms, including its inhibitory effect on pulmonary vasoconstriction (Robertson et al., 2000 Br. J. Pharmacol. 131:5-9; Wang et al., 2001 Am. J. Respir. Cell Mol. Biol. 25:628-635; Nagaoka et al., 2004 Am. J. Physiol. Lung Cell Mol. Physiol. 287:L665-L672), the prevention of mechanical stress-induced expression of growth factors (Wilson et al., 1993 J. Cell Biol. 123:741-747), or the inhibition of Rho kinase-mediated mitogenic effects of serotonin (Liu et al., 2004 Circ. Res. 95: 579-586) and Rho kinase-mediated inhibition of nitric oxide synthase (Takemoto et al., 2002 Circulation 106: 57-62).

Current therapies for pulmonary hypertension are unsatisfactory. These typically involve calcium channel antagonists, prostacyclins, endothelin receptor antagonists and long-term anticoagulant therapy. However, each treatment has limitations and side effects.

Consequently there is a long felt need for a new and combined medicament for the treatment of PAH, preferably employing lower doses of the active agents, which exhibits fewer or no adverse effects (i.e., less toxicity) and a favorable profile in terms of effectiveness in patients in different stages of PAH.

### Summary of the Invention

A therapeutic combination is disclosed herein for the treatment and/or prevention of PAH and/or angina. The disclosed combination comprises an effective amount of the Rho-kinase inhibitor fasudil and the PDE inhibitor sildenafil .

The fasudil may be formulated for administration via inhalation. For example, the fasudil may be formulated in a dry powder or an aerosolizable solution.

A method of treating and/or preventing PAH and/or stable angina is also disclosed. The method comprises administering effective amounts of the therapeutic combination of Claim 1.

### Brief Description of the Drawings

Figure 1 (A, B and C) shows dose response curves of effects of fasudil (A), iloprost (B) and fasudil after iloprost, 10⁻⁶ M (C) on relaxation of vasoconstricted small pulmonary arteries rings.

Figure 2 (A, B and C) shows dose response curves of effects of fasudil (A), bosentan (B) and fasudil after bosentan, 5x10⁻⁵ M (C) on relaxation of vasoconstricted small pulmonary arteries rings..

Figure 3 (A, B and C) shows dose response curves of effects of fasudil (A), Viagra™ (B) and fasudil after Viagra™, 10⁻⁵ M (C) on relaxation of vasoconstricted small pulmonary arteries rings..

### Detailed Description of the Preferred Embodiments

In an embodiment of the present invention, a combination therapy is disclosed for treating and/or preventing PAH. In one preferred embodiment, the combination therapy comprises a therapeutically effective amount of a Rho-kinase inhibitor namely fasudil, in combination with a therapeutically effective amount of a PDE inhibitor, namely sildenafil. The active agents may be formulated and administered together or may be formulated and administered independently. For example the active agents may be formulated together in a single tablet or capsule, a single dry powder formulated for inhalation, or a solution formulated for aerosolization and inhalation. The active agents may be formulated and administered separately. In one preferred embodiment, the Rho-kinase inhibitor is aerosolized.

The additional active agent sildenafil (Viagra®), used in the therapeutic combination modulates pulmonary arterial pressure through a mechanism which is distinct from that of fasudil.

In other preferred embodiments, therapeutic combination is disclosed for treatment and/or prevention of stable angina.

### Rho-kinase inhibitors

Rho-kinase is involved in such processes as tumor invasion, cell adhesion, smooth muscle contraction, and formation of focal adhesion fibers, as revealed using inhibitor Y-27632. Another Rho-kinase inhibitor, 1-(5-isoquinolinesulfonyl)-homopiperazine (HA-1077 or Fasudil), is currently used in the treatment of cerebral vasospasm; the related nanomolar inhibitor H-1152P improves on its selectivity and potency. It was recently found that RhoA/Rho kinase signaling is involved in both vasoconstriction and vascular remodeling in the mouse model of hypoxic pulmonary hypertension (Fagan L.A. et al. 2004 Am. J. Physiol. Lung Cell Mol. Physiol. 287:L656-L664), and that Rho kinase-mediated vasoconstriction substantially contributes to the sustained elevation of pulmonary vascular resistance in rat model of hypoxic pulmonary hypertension (Nagaoka et al., 2004 Am. J. Physiol. Lung Cell Mol. Physiol. 287:L665-L672). In the latter study, the acute effect of intravenous Y-27632, a selective Rho-kinase inhibitor, in chronically hypoxic rats was striking (i.e., it nearly normalized the elevated pulmonary artery pressure). However, intravenous Y-27632 had no pulmonary vascular selectivity and also caused systemic vasodilation. Uehata et al. (1997, Nature 389:990-994) have reported that oral administration of Y-27632 has potent hypotensive effects in rat models of systemic hypertension, but has a smaller transient effect in normotensive rats. Nagaoka et al. (2005 Am. J. Respir. Crit. Care Med. 171:494-499) reported that 5 minutes of inhaled Y-27632 decreased mean pulmonary arterial pressure without reducing mean systemic arterial pressure. The hypotensive effect of inhaled Y-27632 on hypoxic pulmonary hypertension was greater that that of inhaled nitric oxide, and the effect lasted for at least 5 hrs. Fasudil (Asahi Kasei Pharma Co., Tokyo, Japan) is also a Rho-kinase inhibitor that is metabolized in the liver to a more specific Rho-kinase inhibitor, hydroxyfasudil, after oral administration in vivo (Shimokawa H. et al. 1999 Cardiovasc. Res. 43:1029-1039). Inhaled fasudil caused selective mean pulmonary arterial pressure reductions in monocrotaline-induced pulmonary hypertension and in spontaneous PH in rats, as well as in chronically hypoxic rats. In patients with severe pulmonary hypertension, fasudil, administered intravenously, significantly reduced pulmonary vascular resistance without side effects (Fukumoto Y. et al. 2005 Heart 91:391-392).

Improved HA-1077 analog, (S)-(+)-2-methyl-1-[(4-methyl-5-isoquinoline)sulfonyl]-homopiperazine (H-1152P), which is a more selective inhibitor of Rho-kinase, with a K(i) value of 1.6 nM for Rho-kinase was synthesized by Sasaki et al. (2002 Pharmacol Ther. 93:225-32). Additional specific Rho-kinase inhibitors have been synthesized by Tamura et al. (2005 Biochim. Biophys. Acta*).*

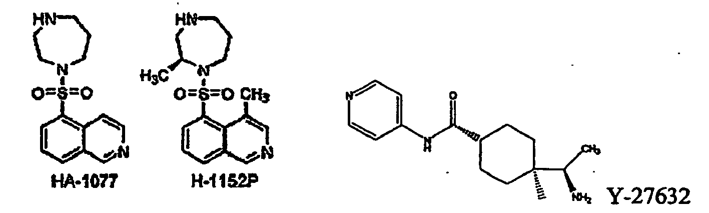

The role of Rho-kinase in cardiovascular medicine and in the treatment of stable angina is described by Shimokawa and Takeshita 2005 (Arterioscler Thromb Vasc Biol 25:X-X) and Hirooka and Shimokawa 2005 (Am J Cardiovasc Drugs 5(1): 31-39).

HMG-CoA reductase inhibitors, known as statins, have been shown to suppress Rho-kinase activity in some systems. See e.g., Tramontano et al. 2004 (Biochem Biophys Res Comm 320: 34-38) and Ohnaka et al. 2001 (Biochem Biophys Res Comm 287: 337-342). Accordingly, statins such as fluvastatin, pitavastatin, pravastatin, atorvastatin, etc., may be useful in potentiating or otherwise advantageously modulating the therapeutic effect of the Rho-kinase inhibits disclosed herein.

### BACKGROUND ART

### Epoprostenol Derivatives

A continuous infusion of prostacyclin (Flolan®, GlaxoSmithKline) was the first therapy shown to reduce mortality in a controlled study of patients with severe pulmonary hypertension. However, its use is associated with a number of serious drawbacks (Barst R.J. et al. 1996 N Engl J Med 334:296-301; Badesch D.B. et al. 2000 Ann Intern Med 132:425-434). The lack of pulmonary selectivity results in systemic side effects, tolerance leads to progressive increases in the dose, and there may be recurrent infections of the intravenous catheter. As an alternative, inhaled nitric oxide possesses pulmonary selectivity, but it is less potent than prostacyclin in the pulmonary vasculature. Moreover, an interruption in the inhalation of continuous nitric oxide may cause rebound pulmonary hypertension. Designed to combine the beneficial effects of prostacyclin with those of an inhalational application, aerosolized prostacyclin was found to be a potent pulmonary vasodilator in patients with acute respiratory failure, exerting preferential vasodilatation in well-ventilated lung regions (Walmrath D. et al. 1993 Lancet 342:961-962; Walmrath D. et al. 1995 Am J Respir Crit Care Med 151:724-730; Walmrath D. et al. 1996 Am J Respir Crit Care Med 153:991-996; Zwissler B. et al. 1996 Am J Respir Crit Care Med 154:1671-1677). Similar results were obtained in spontaneously breathing patients who had lung fibrosis and severe pulmonary hypertension (Olschewski H. et al. 1999 Am JRespir Crit Care Med 160:600-607).

Three epoprostenol analogs have been studied in the treatment of PAH: treprostinol (Remodulin®, United Therapeutics), beraprost, and iloprost. Treprostinol is a stable analogue of epoprostenol, which is given continuously subcutaneously. Escalation of dosage has been limited by significant infusion site pain. Thus many patients do not receive therapeutic doses. Beraprost is active orally and has shown a benefit in a study in PAH at 3 and 6 months but not at 9 or 12 months (Barst, R.J. 2003 J. Am. Coll. Cardiol. 41:2119-25. Iloprost can be given intravenously or by nebulizer. The advantages of the nebulizer method of delivery is that less of the substance reaches the systemic circulation (a "pseudoselective" pulmonary vasodilator). Iloprost is generally given six to nine times a day, which may disrupt the patient's lifestyle; dosing frequency may be reduced by combining iloprost with an agent having a therapeutic effect on the pulmonary hypertension through a different mechanism and possibly acting synergistically.

Recently Abe K. et al. (2005 J Cardiovasc Pharmacol 45:120-124) demonstrated that prostacyclin and beraprost lack the inhibitory effect on Rho-kinase, when administered to rats, indicating that a combination therapy with prostacyclin and a Rho-kinase inhibitor could have further beneficial effects on puhnonary hypertension.

### Iloprost

Iloprost (see US 4,692,464) is a stable analogue of prostacyclin that is associated with a longer duration of vasodilatation (Fitscha P. et al. 1987 Adv Prostaglandin Thromboxane Leukot Res 17:450-454). When administered by aerosolization to patients with pulmonary hypertension, its pulmonary vasodilative potency was similar to that of prostacyclin, but its effects lasted for 30 to 90 minutes, as compared with only 15 minutes for the prostacyclin (Hoeper M.M. et al. 2000 J Am Coll Cardiol 35:176-182; Olschewski H. et al. 1999 Am J Respir Crit Care Med 160:600-607; Olschewski H. et al. 1996 Ann Intern Med 124:820-824; Gessler T. et al. 2001 Eur Respir J 17:14-19; Wensel R. et al. 2000 Circulation 101:2388-2392). Several open-label, uncontrolled studies of patients with severe pulmonary hypertension suggested that long-term use of aerosolized iloprost results in substantial clinical improvement (Olschewski H. et al. 1999 Am J Respir Crit Care Med 160:600-607; Olschewski H. et al. 1996 Ann Intern Med 124:820-824; Hoeper M.M. et al. 2000 N Engl J Med 342:1866-1870; Olschewski H. et al. 1998 Intensive Care Med 24:631-634.; Stricker H. et al. 1999 Schweiz Med Wochenschr 129:923-927; Olschewski H. et al. 2000 Ann Intern Med 132:435-443; Beghetti M. et al. 2001 Heart 86:E10-E10). A multi-center randomized placebo controlled study of patients with severe PAH has demonstrated improved exercise capacity in patients receiving iloprost versus those receiving placebo (Olschewski H et al 2002 NEJM 345:322-9).

### Sarpogrelate

Sarpogrelate ((φ-amino-alkoxy)phenyl-ethylethylbenzene) is a serotonin blocker, and more particularly, a selective 5-hydroxytryptamine receptor subtype 2A (5-HT_{2A}) antagonist. It is metabolized to racemic M-1 and both enantiomers of M-1 are also antagonists of 5-HT_{2A} receptors. Sarpogrelate inhibits responses to 5-HT mediated by 5-HT_{2A} receptors such as platelet aggregation, vasoconstriction and vascular smooth muscle proliferation. Sarpogrelate is efficacious in animal models of thrombosis, coronary artery spasm, atherosclerosis, restenosis, peripheral vascular disease, pulmonary hypertension, ischemic heart disease, myocardial infarction, diabetes and kidney disease (Doggrell S.A. 2004 Expert Opin. Investing. Drugs 13:7865-874). In the commonly used model of pulmonary hypertension, induced by monocrotaline in rat, the severity of pulmonary hypertension, determined by the medial thickness of the small pulmonary arteries and right ventricle/left ventricle and septum ration, were reduced by treatment with sarpogrelate (Myata M. et al. 2000 Lung 178:63-73). Sarpogrelate also reduced the thickening of the alveolar walls and interstitial inflammatory cell infiltration. The number of proliferating cell nuclear antigen-positive cells was also reduced by sarpogrelate (Myata M. et al. 2000 Lung 178:63-73).

The benefit of sarpogrelate in the monocrotaline model of pulmonary hypertension has been confirmed. Sarpogrelate, immediately following monocrotaline injection, suppressed the severe pulmonary vascular remodeling and right side heart failure and reduced mortality (Hironaka E. et al. 2003 Cardiovasc. Res. 60:692-629). However, late treatment with sarpogrelate failed to reverse established pulmonary hypertension. Sarpogrelate was also tested in a rat model of pulmonary embolism/pulmonary hypertension. In that model, rats injected with IL-6 develop extensive microarterial thrombosis in the lungs along with hypercoagulation and hyperfibrinolysis. The simultaneous sarpogrelate treatment in the rat prevented the IL-6-induced increase in medial thickness of small pulmonary arteries and the right ventricular hypertrophy (Myata M. et al. 2001 Chest 119:554-561; Saini H.K. et al. 2004 Caardiovasc. Drug Rev. 22:27-54). Sarpogrelate has no effect on the systolic blood pressure or heart weight of spontaneously hypertensive rats (Setoguchi Y. et al. 2002 Pharmacol. 64:71-75).

In clinical trials, sarpogrelate significantly decreased respiratory failure and mean pulmonary arterial pressure in patients with Raynaud's phenomenon and systemic sclerosis (Kato S. et al. 2000 Respirology 5:27-32; Kato S. et al. 2000 J. Int. Med. Res. 28:258-268).

In addition, sarpogrelate has also been reported as an effective treatment for stable angina. Kinugawa et al., (Am Heart J 2002;144:e1) had previously demonstrated that a single oral administration of sarpogrelate, a 5-HT2A receptor antagonist, may improve exercise capacity in anginal patients with well-developed collaterals. The researchers further investigated the effectiveness of 2-week treatment with sarpogrelate on anginal symptoms and exercise capacity in anginal patients.

A treadmill exercise test was repeated after a 2-week period with or without sarpogrelate (100 mg 3 times a day) in 20 patients with angiographically proven stable angina. Anginal symptoms and daily physical activity by the specific activity scale (SAS) were also evaluated. Treatment with sarpogrelate significantly increased the SAS score and prolonged exercise time to the onset of 0.1-mV ST depression. When data were analyzed in a subgroup of patients (n = 8) with well-developed collaterals, the treatment with sarpogrelate decreased the number of anginal attacks (control vs sarpogrelate, 3.0 ± 2.8 vs 0.9 ± 1.1/2 weeks, P < .05), increased the SAS score (5.2 ± 1.6 vs 6.2 ± 1.3 METS, P < .05), and increased the time to the onset of 0.1-mV ST depression (235 ± 84 vs 295 ± 127 seconds, P < .05). In addition, the double product at the onset of 0.1-mV ST depression increased by 15% (P < .05) after sarpogrelate. In contrast, all parameters were not significantly changed after sarpogrelate treatment in patients (n = 12) without well-developed collaterals. These findings indicate the therapeutic effectiveness of sarpogrelate for anginal patients, especially for patients with well-developed collaterals.

### Endothelin Receptor Antagonists (ETRA)

There is increasing evidence that endothelin-1 has a pathogenic role in pulmonary arterial hypertension and that blockade of endothelin receptors may be beneficial. Endothelin-1 is a potent endogenous vasoconstrictor and smooth-muscle mitogen that is overexpressed in the plasma and lung tissue of patients with pulmonary arterial hypertension. There are two classes of endothelin receptors: Endothelin A, ET-A and Endothelin B, ET-B receptors, which play significantly different roles in regulating blood vessel diameter. The binding of endothelin to ET-A receptors located on smooth muscle cells causes vasoconstriction, whereas the binding of endothelin to ET-B receptors located on the vascular endothelium causes vasodilatation through the production of nitric oxide. This latter activity of the ET-B receptor is thought to be counter-regulatory and protects against excessive vasoconstriction.

Therefore, another attractive approach to treating pulmonary hypertension has been the blockade of these endothelin receptors. Two types of ETRAs have been developed: dual ETRAs, which block the receptors for both ET-A and ET-B, and selective ETRAs, which block only the ET-A receptor.

### a) Dual Endothelin Receptor Antagonist

The first generation ETRAs are non-selective and block both the ET-A and ET-B receptors. Bosentan (Tracleer™) is the first FDA approved ETRA (see US 5,292,740). Two placebo controlled trials of bosentan (an endothelin receptor A and B antagonist) have been conducted (Channick RN. et al. 2001 Lancet 358:1119-1123; Rubin L.J. et al. 2002 N Engl J Med 346:896-903). The six minute walk test improved in the whole group, but the improvement was greater when the drug was used in higher doses. However, liver toxicity occurred with the higher dose.

### b) Selective Endothelin Receptor Antagonist

Second generation ETRAs bind to the ET-A receptor in preference to the ET-B receptor. Currently, there are two selective ETRAs in clinical trials: sitaxsentan and ambrisentan (BSF 208075). A pure endothelin A antagonist, sitaxsentan has been used in an open pilot study. This showed an improvement in the six minute walk test and a decrease in pulmonary vascular resistance of 30% (Barst R.J. et al. 2000 Circulation 102:II-427).

A more potent endothelin compound, TBC3711 (Encysive Pharmaceuticals), entered Phase I testing in December 2001. This drug holds potential for treating chronic heart failure and essential hypertension.

There are small clinical trials of using bosentan in patients that are already on other medications for the treatment of pulmonary hypertension (Hoeper M.M. et al. 2003 in: "Pulmonary Hypertension: Clinical", Abstr. A275, May 18, 2003; Pulmonary Hypertension Roundtable 2002, Phassociation.org/medical/advances in PH/spring 2002).

### Nitric oxide production

Endothelial production of nitric oxide is diminished with pulmonary hypertension, prompting attempts to reverse this defect either by giving continuous inhaled nitric oxide, which is effective but difficult to administer, or by increasing the substrate for nitric oxide L-arginine (Nagaya N. et al. 2001 Am J Respir Crit Care Med 163:887-891). A trial of supplementation with L-arginine is currently under way.

### PDE Inhibitors

In addition to increasing the supply of nitric oxide, attempts to directly increase cyclic nucleotide second messenger levels in the smooth muscle cells have been made. Sildenafil used for erectile dysfunction blocks the enzyme phosphodiesterase type 5 present in the corpus cavernosum of the penis and also the lungs. This raises the possibility that a phosphodiesterase inhibitor, preferably a PDE type 5 inhibitor such as sildenafil, could be a relatively selective pulmonary vasodilator. There is empirical evidence supporting the inventor's selection of PDE inhibitors as a target compound in a combination therapy (see e.g., Michelakis E. et al. 2002 Circulation 105:2398-2403; Ghofrani H. et al. 2002 Lancet 360:895-900).

Although aerosolized prostacyclin (PGI₂) has been suggested for selective pulmonary vasodilation as discussed above, its effect rapidly levels off after termination of nebulization. Stabilization of the second-messenger cAMP by phosphodiesterase (PDE) inhibition has been suggested as a strategy for amplification of the vasodilative response to nebulized PGI₂. Lung PDE3/4 inhibition, achieved by intravascular or transbronchial administration of subthreshold doses of specific PDE inhibitors, synergistically amplified the pulmonary vasodilatory response to inhaled PGI₂, concomitant with an improvement in ventilation-perfusion matching and a reduction in lung edema formation. The combination of nebulized PGI₂ and fasudil with PDE3/4 inhibition may thus offer a new concept for selective pulmonary vasodilation, with maintenance of gas exchange in respiratory failure and pulmonary hypertension (Schermuly R.T. et. al. 2000 J Pharmacol Exp Ther 292:512-20). There are some reports of small clinical studies showing that such combination therapy may be efficacious in the treatment of pulmonary hypertension (Ghofrani et al. 2002 Crit Care Med 30:2489-92; Ghofrani et al. 2003 J Am Coll Cardiol 42:158-164; Ghofrani et al. 2002 Ann Intern Med 136:515-22).

Isozymes of cyclic-3', 5'-nucleotide phosphodiesterase (PDE) are a critically important component of the cyclic-3',5'-adenosine monophosphate (cAMP) protein kinase A (PKA) signaling pathway. The superfamily of PDE isozymes consist of at least nine gene families (types): PDE1 to PDE9. Some PDE families are very diverse and consist of several subtypes and numerous PDE isoform-splice variants. PDE isozymes differ in molecular structure, catalytic properties, intracellular regulation and location, and sensitivity to selective inhibitors, as well as differential expression in various cell types.

A phosphodiesterase (PDE) inhibitor is defined herein as any drug used in the treatment of pulmonary arterial hypertension that works by blocking the inactivation of cyclic AMP. There are five major subtypes of phosphodiesterase (PDE); the drugs enoximone (inhibits PDE IV) and milrinone (Primacor®) (inhibits PDE IIIc) are most commonly used medically. Other phosphodiesterase inhibitors include Amrinone (Inocor®) used to improve myocardial function, pulmonary and systemic vasodilation, and sildenafil (Viagra®), tadalafil (Cialis®) and vardenafil (LEVTTRA®) - selective phosphodiesterase V inhibitors.

### Calcium Channel Blockers

Calcium channel blockers, or antagonists, act by blocking the entry of calcium into muscle cells of heart and arteries so that the contraction of the heart decreases and the arteries dilate. With the dilation of the arteries, arterial pressure is reduced so that it is easier for the heart to pump blood. This also reduces the heart's oxygen requirement. Calcium channel blockers are useful for treating PPH. Due to blood pressure lowering effects, calcium channel blockers are also useful to treat high blood pressure. Because they slow the heart rate, calcium channel blockers may be used to treat rapid heart rhythms such as atrial fibrillation. Calcium channel blockers are also administered to patients after a heart attack and may be helpful in treatment of arteriosclerosis.

Calcium channel blockers include amlodipine (US 4,572,909); bepridil (US 3,962,238); clentiazem (US 4,567,175); diltiazem (US 3,562,257); fendiline (US 3,262,977); gallopamil (US 3,261,859); mibefradil (US 4,808,605); prenylamine (US 3,152,173); semotiadil (US 4,786,635); terodiline (US 3,371,014); verapamil (US 3,261,859); aranidipine (US 4,446,325); bamidipine (US 4,220,649): benidipine (European Patent Application Publication No. 106,275); cilnidipine (US 4,672,068); efonidipine (US 4,885,284); elgodipine (US 4,952,592); felodipine (US 4,264,611); isradipine (US 4,466,972); lacidipine (US 4,801,599); lercanidipine (US 4,705,797); manidipine (US 4,892,875); nicardipine (US 3,985,758); nifedipine (US 3,485,847); nilvadipine (US 4,338,322); nimodipine (US 3,799,934); nisoldipine (US 4.154,839); nitrendipine (US 3,799,934); cinnarizine (US 2,882,271); flunarizine (US 3,773,939); lidoflazine (US 3,267,104); lomerizine (US .4,663,325); bencyclane (Hungarian Patent No. 151,865); etafenone (German Patent No. 1,265,758); and perhexiline (British Patent No. 1,025,578).

The compounds can be present as pharmaceutically acceptable salts. If these compounds have, for example, at least one basic center, they can form acid addition salts. Corresponding acid addition salts can also be formed having, if desired, an additionally present basic center. The compounds having at least one acid group (for example COOH) can also form salts with bases. Corresponding internal salts may furthermore be formed, if a compound of formula comprises e.g., both a carboxy and an amino group.

### Gleevec

Recently, Gleevec (Imatinib) has been shown to be successfully used in two well established experimental models of progressive pulmonary arterial hypertension. It was found that the treatment resulted in virtually complete reversal of lung vascular remodeling, pulmonary hypertension and right heart hypertrophy.

In the Sept. 29, 2005 issue of the New England Journal of Medicine, a 61-year-old man suffering from an advanced case of the disease saw his condition improve and stabilize after taking Gleevec (imatinib) -- even though all other medications had failed. "Only the addition of Gleevec was able to prevent further deterioration, and even improved his condition," said co-researcher Dr. Hossein A. Ghofrani, of University Hospital Giessen, in Germany. Although a single case report does not warrant widespread use of Gleevec for pulmonary hypertension, the German researchers who wrote the report said they are now planning a large clinical trial.

The therapeutic combination disclosed herein for the treatment and/or prevention of PAH and/or stable angina is a combination of known drugs. The pharmacologic properties of these drugs are well known to the skilled practitioner. The combinations may be formulated or otherwise individually administered such that the relative amounts of each drug in the combination is sufficient, when combined to treat and/or prevent at least one symptom associated with PAH and/or stable angina. The symptoms of PAH and stable angina are well known. Moreover, animal models of these conditions may be used to optimize dosages (see e.g., the references cited and incorporated herein). The skilled practitioner will be able to determine without undue experimentation optimal dosages.

It is understood that the drugs disclosed for use in the combination therapies may be used in their free forms or as salts thereof.

The drugs in the recited therapeutic combinations can be administered, as described herein, according to any of a number of standard methods including, but not limited to injection, suppository, nasal spray, time-release implant, transdermal patch, and the like. In particularly preferred embodiments, at least one of the drugs in the combination is administered orally (e.g. as a syrup, capsule, or tablet) or by inhalation (either as a dry powder formulation or as a nebulized formulation). In one preferred embodiment, fasudil, or salts thereof, may be given orally. More preferably, the fasudil, or salts thereof, is administered as a sustained-release formulation sufficient to provide extended plasma half-life and minimize the peaks and troughs of plasma concentrations between dosing. Such formulations are well known in the art and may enhance bioavailability of the drug. In another preferred embodiment, fasudil, or salts thereof, may be administered by inhalation.

More generally, injectable preparations include sterile suspensions, solutions or emulsions of the active ingredients in aqueous or oily vehicles. The compositions may also contain formulating agents, such as suspending, stabilizing and/or dispersing agent. The formulations for injection may be presented in unit dosage form, e.g., in ampules or in multidose containers, and may contain added preservatives.

Alternatively, the injectable formulation may be provided in powder form for reconstitution with a suitable vehicle, including but not limited to sterile pyrogen free water, buffer, dextrose solution, etc., before use. To this end, stored preparations can be supplied in unit dosage forms and reconstituted prior to use in vivo.

For prolonged delivery, the active ingredients can be formulated as a depot preparation, for administration by implantation; e.g., subcutaneous, intradermal, or intramuscular injection. Thus, for example, the active ingredient may be formulated with suitable polymeric or hydrophobic materials (e.g., as an emulsion in an acceptable oil) or ion exchange resins, or as sparingly soluble derivatives; e.g., as a sparingly soluble salt form of the candidate drugs.

Alternatively, transdermal delivery systems manufactured as an adhesive disc or patch which slowly releases the active ingredient for percutaneous absorption may be used. To this end, permeation enhancers may be used to facilitate transdermal penetration of the active ingredients.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

For buccal administration, the compositions may take the form of tablets or lozenges formulated in conventional manner. For rectal and vaginal routes of administration, the active ingredients may be formulated as solutions (for retention enemas) suppositories or ointments.

For administration by inhalation, the active ingredients can be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compositions may, if desired, be presented in a pack or dispenser device which may contain one or more unit dosage forms containing the active ingredients. The pack may for example comprise metal or plastic foil, such as a blister pack. The pack or dispenser device may be accompanied by instructions for administration.

### EXAMPLE 1

### Isolated PA rings

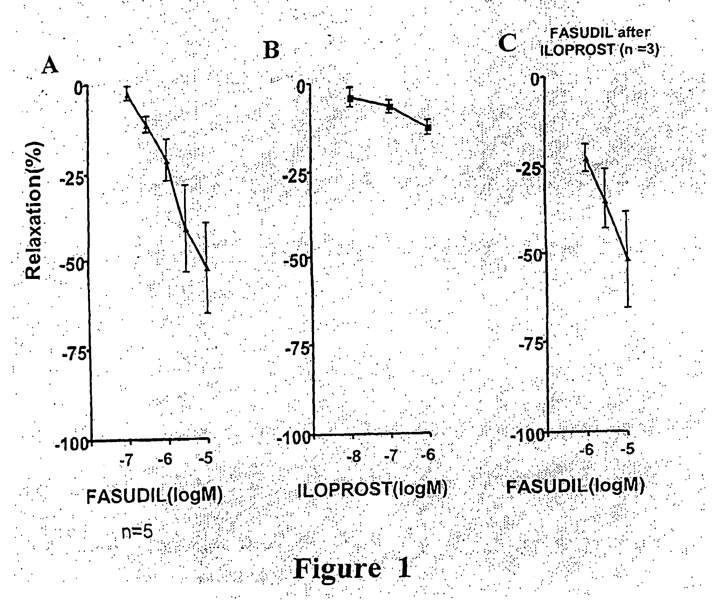

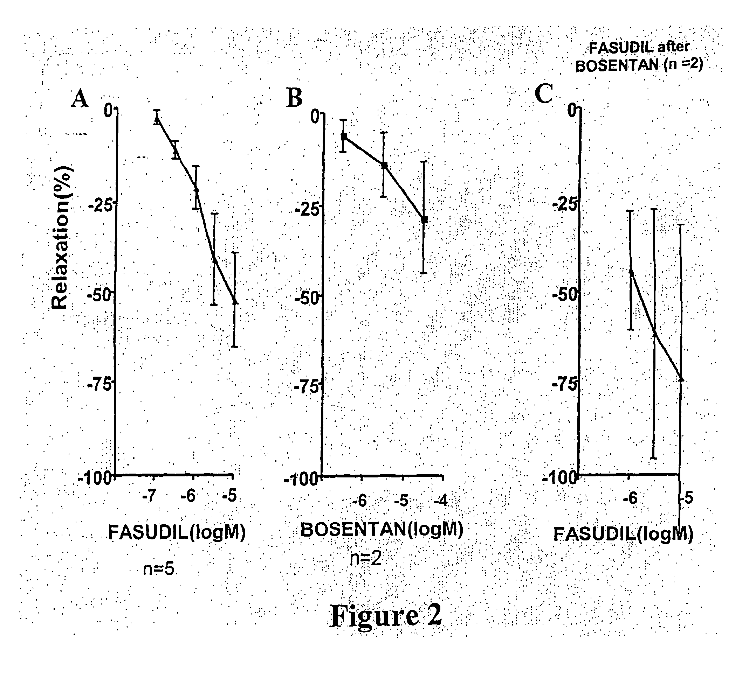

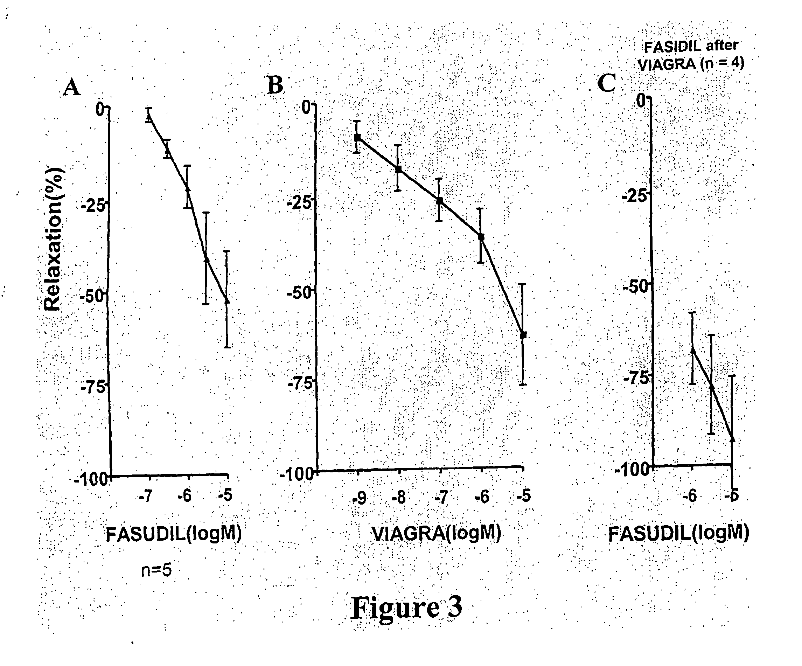

Small (4th or 5th branch) pulmonary arteries (SPA, 200-300 µm ID) were isolated from rats exposed to chronic hypoxia (∼10% oxygen) for 3 to 4 weeks. Briefly, after anesthesia with pentobarbital sodium (30 mg ip) and heparinization (100 IU), the heart and lungs were removed *en bloc,* and the SPA were isolated under a dissecting microscope. Care was taken to avoid damage to the endothelium. The SPA rings were placed on steel wires attached to a force transducer and suspended in baths containing 10 ml of physiological salt solution (PSS) at 37°C. Resting passive force was adjusted to a previously determined optimal tension (determined by maximum response to 80 mM KCl: 400 mg for SPA from normotensive lungs (NL), and 750 mg for SPA from hypoxic hypertensive lungs (HL). Rings were gassed with 21% O₂-5% CO₂-74% N₂ and allowed to equilibrate for 60 min. The artery rings were then constricted with a combination of endothelin-1 (ET-1) (3 nM) plus thromboxane analogue U46619 (30 nM) plus 5-HT (3 µM). After the vasoconstrictor-induced constriction of the pulmonary artery rings had reached a plateau, the various vasodilators (iloprost, bosentan, sildenafil (Viagra™) or fasudil) were added individually to the muscle baths to achieve a calculated concentration. When the vasodilator response to the first concentration of drug had reached a plateau, additional vasodilator was added to the bath to achieve the next higher concentration, and so on until the highest concentration used was reached to obtain dose-response curves for each compound individually. Then, after the highest concentrations of iloprost, bosentan, and sildenafil were tested, increasing concentrations of fasudil were added sequentially to the same baths to obtain dose-response curves for fasudil in the presence of the tested compounds. Figures 1-3 show the obtained dose-response curves.

### EXAMPLE 2

### Isolated PA rings

The experiments are performed essentially as described above, but the rings are constricted with each of the vasoconstrictors individually. The dose response curves of fasudil with iloprost, fasudil with bosentan, fasudil with sildenafil show synergistic effects on the relaxation of the PA rings.

### EXAMPLE 3

### Conscious Catheterized Rats

Experiments are performed with a chronically hypoxic, pulmonary-hypertensive group of rats which have been exposed to hypobaric hypoxia (410 mmHg barometric pressure, 76 mmHg inspired O₂ tension) for 3-4 wk in a chamber flushed continuously with room air to prevent accumulation of CO₂, NH₃, and H₂O. Hypobaric exposure has been 24 h/day, except when the chamber has been opened for 10-15 min every 2 days to remove rats or clean cages and replenish food and water. All rats are exposed to a 12:12-h light-dark cycle and allowed free access to standard rat food and water.

The chronically hypoxic rats are anesthetized with ketamine (100 mg/kg) and xylazine (15 mg/kg) for placement of catheters in the right jugular vein and pulmonary and right carotid arteries (Oka M. 2001 Am J Physiol Lung Cell Mol Physiol 280: L432-L435). The rats are allowed to recover for 48 h in room air. After recovery, conscious rats are placed in a ventilated plastic box, and pulmonary and systemic arterial pressures are measured with pressure transducers. Cardiac output is determined by a standard dye-dilution method, and total pulmonary resistance (TPR) is calculated by dividing mean PA pressure (MPAP) by cardiac output. The rats are then injected with various doses of vasodilators, such as iloprost, bosentan or sildenafil alone or in combination with fasudil at 30-min intervals between doses. Pressures are measured 10 min after injection of each dose. Cardiac output measurements are repeated 10 min after injection of the lowest and the highest doses tested for each compound. After the final dose, rats are again exposed to 10 min of hypoxia. The combinations show synergistic effects on the MPAP and TPR of the chronically hypoxic rats.

### EXAMPLE 4

### Isolated Perfused Lungs

After pulmonary-hypertensive rats are removed from the hypobaric chamber and anesthesized with pentobarbital sodium (30 mg i.p.), lungs are isolated and perfused for vasoreactivity studies. The techniques of lung isolation, ventilation, and perfusion have been described in detail elsewhere (Morio Y. & McMurtry I.F. 2002 J Appl Physiol 92:527-534). The perfusate is 20 ml of PSS. Ficoll (4 g/100 ml; type 70, Sigma) is included as a colloid, and 3.1 µM meclofenamate (Sigma) is added to inhibit synthesis of vasodilator prostaglandins. Perfusion rate is 0.04 ml·g body wt⁻¹·min⁻¹. PSS-perfused hypoxic hypertensive lungs (HL) are equilibrated at 37°C for 20 min during ventilation with 8% O₂-5% CO₂-87% N₂. After equilibration, two hypoxic pressor responses are elicited by 10 min of ventilation with 0% O₂-5% CO₂-95% N₂ and 3% O₂-5% CO₂-92% N₂, separated by 10 min of normoxic ventilation, to induce hypoxic vasoreactivity. The drugs are added to the perfusate reservoir to achieve the calculated circulating concentrations. The vasoconstriction is achieved by Nomega-nitro-L-arginine (L-NNA), a NO synthase (NOS) inhibitor. After development of the L-NNA-induced vasoconstriction, iloprost, bosentan, sildenafil, or fasudil alone, or iloprost, bosentan, sildenafil each in combination with fasudil are added cumulatively to the perfusate of separate lungs at 10-min intevals. The baseline perfusion pressure is measured before and after the vasodilators. The combinations of the tested drugs showed synergistic effects on the reduction of the baseline perfusion pressure.

### References

1. Kato et al. (2000) Respirology 5: 27-32.
2. Kato et al. (2000) J. Int. Med. Res. 28: 258-268.
3. Doggrell (2004) Expert Opin. Investig. Drugs 13: 7865-874.
4. Saini et al (2004) Cardiovasc. Drug Rev. 22: 27-54.
5. Abe et al. (2005) J Cardiovasc Pharmacol 45:120-124.
6. Nagaoka et al. (2004) Am. J. Physiol. Lung Cell Mol. Physiol. 287: L665-L672.
7. Fukumoto et al. (2005) Heart 91: 391-392.
8. Takemoto et al. (2002) Circulation 106: 57-62.
9. Abe et al. (2004) Circ Res 94: 385-393.
10. Tramontano et al. (2004) Biochem Biophys Res Comm 320: 34-38.
11. Ohnaka et al. (2001) Biochem Biophys Res Comm 287: 337-342.
12. Shimokawa et al. (2005) Cardiovasc. Res. 43:1029-1039**.**
13. Hirooka et al. (2005) Am J Cardiovasc Drugs 5(1): 31-39.

## Claims

1. A therapeutic combination, comprising an effective amount of fasudil and sildenafil.

2. The therapeutic combination of Claim 1, wherein fasudil and sildenafil may be formulated together or independently.

3. The therapeutic combination of Claims 1 or 2, wherein the fasudil is formulated for administration via inhalation.

4. The therapeutic combination of Claim 3, wherein the fasudil is formulated in a dry powder or an aerosolizable solution.

5. A therapeutic combination, comprising fasudil and sildenafil, for use in the treatment and/or prevention of at least one symptom associated with PAH.

6. A medicament, comprising the therapeutic combination according to any one of Claims 1 to 5, for use in the treatment and/or prevention of PAH and/or stable angina.

## Patentansprüche

1. Therapeutische Kombination, umfassend eine wirksame Menge an Fasudil und Sildenafil.

2. Therapeutische Kombination nach Anspruch 1, wobei Fasudil und Sildenafil zusammen oder unabhängig formuliert sein können.

3. Therapeutische Kombination nach Anspruch 1 oder 2, wobei das Fasudil zur Verabreichung durch Inhalation formuliert ist.

4. Therapeutische Kombination nach Anspruch 3, wobei das Fasudil als ein trockenes Pulver oder als eine aerosolisierbare Lösung formuliert ist.

5. Therapeutische Kombination, umfassend Fasudil und Sildenafil, zur Verwendung bei der Behandlung und/oder Prävention von mindestens einem Symptom, das mit PAH verbunden ist.

6. Medikament, umfassend die therapeutische Kombination nach einem der Ansprüche 1 bis 5, zur Verwendung bei der Behandlung und/oder Prävention von PAH und/oder stabiler Angina.

## Revendications

1. Combinaison thérapeutique, comprenant une quantité efficace de fasudil et de sildénafil.

2. Combinaison thérapeutique selon la revendication 1, dans laquelle le fasudil et le sildénafil peuvent être formulés ensemble ou indépendamment.

3. Combinaison thérapeutique selon la revendication 1 ou 2, dans laquelle le fasudil est formulé pour une administration par inhalation.

4. Combinaison thérapeutique selon la revendication 3, dans laquelle le fasudil est formulé dans une poudre sèche ou une solution aérosolisable.

5. Combinaison thérapeutique, comprenant du fasudil et du sildénafil, pour une utilisation dans le traitement et/ou la prévention d'au moins un symptôme associé au PAH.

6. Médicament, comprenant la combinaison thérapeutique selon l'une quelconque des revendications 1 à 5, pour une utilisation dans le traitement et/ou la prévention du PAH et/ou de l'angine stable.
